(19) Europäisches Patentamt

European Patent Office

Office européen des brevets

(11) **EP 0 387 793 B1**

(12) **EUROPÄISCHE PATENTSCHRIFT**

(45) Veröffentlichungstag und Bekanntmachung des Hinweises auf die Patenterteilung:
**07.01.1998 Patentblatt 1998/02**

(51) Int. Cl.$^6$: **A61B 5/00**, G01N 21/35

(21) Anmeldenummer: **90104729.0**

(22) Anmeldetag: **13.03.1990**

(54) **Verfahren und Anordnung zur Darstellung von Strukturen**

Process and means for representation of structures

Procédé et dispositif pour présentation de structures

(84) Benannte Vertragsstaaten:
**AT CH DE FR GB IT LI NL SE**

(30) Priorität: **13.03.1989 DD 326506**
**16.11.1989 DD 334606**

(43) Veröffentlichungstag der Anmeldung:
**19.09.1990 Patentblatt 1990/38**

(73) Patentinhaber:
**Krauss, Manfred, Prof., Dr.-Ing.habil.**
**D-09126 Chemnitz (DE)**

(72) Erfinder:
• **Waldmann, Jürgen, Prof.Dr.sc.techn.**
**DDR-1143 Berlin (DD)**
• **Krauss, Mannfred, Prof.Dr.-Ing.habil.**
**DDR-9026 Karl-Marx-Stadt (DD)**
• **Bilz, Dietrich, Dr.sc.med.**
**DDR-1136 Berlin (DD)**
• **Mende, Mattias, Dr.-Ing.**
**DDR-9250 Mittweida (DD)**
• **Kriegel, Bernd, Dipl.-Phys.**
**DDR-1170 Berlin (DD)**

(74) Vertreter:
**Kruspig, Volkmar, Dipl.-Ing. et al**
**Patentanwälte**
**Meissner, Bolte & Partner**
**Postfach 86 06 24**
**81633 München (DE)**

(56) Entgegenhaltungen:
WO-A-88/01485        GB-A- 2 075 668
US-A- 3 674 008      US-A- 4 796 636

## Beschreibung

Die Erfindung betrifft ein Verfahren und eine Anordnung zur Untersuchung von biologischen und technischen Volumenstrukturen unter Nutzung örtlich und volumendefinierter Streulichtmessung.

Verfahren und Anordnung dienen der Untersuchung von Stoffen bzw. Mikro- und Makrostrukturen biologischer Gewebe und Organe mit darin ablaufenden physikalischen und / oder chemischen Prozessen einschließlich des Volumenflusses von Suspensionen in Gefäßen kapillaren Innendurchmessers mittels relativ geringen Lichtintensitäten. Die Erfindung läßt sich somit in Medizin und Biologie sowie in Natur und Technik zur Beobachtung bzw. Beurteilung von Strukturen, Oberflächen, Volumina und Volumenflüssen in natürlichen Stoffen einsetzen.

Es ist bei der Fotoplethysmografie bekannt, konstantes Licht, auch des nahen Infrarotbereiches, in ein Gewebevolumen zu leiten und das aus dem Gewebe austretende Licht als vom Gewebevolumen zurückgestrahltes Licht zu messen bzw. darzustellen. Biophysikalisch betrachtet wird meßtechnisch bei der Fotoplethysmografie ein konstantes Lichtpotential im Gewebe gesetzt, das durch die biologische Information, z.B. durch den roten Blutfarbstoff Hämoglobin, zeitlich und örtlich moduliert wird. Diese Modulation wird in Form eines Transmissionssignals oder eines Rückstreusignals gemessen, wobei sich Strahler und Empfänger einander gegenüber oder in einer Ebene befinden (Perinatal Medicine 4 th European Congress of Perinatal Medicine Prague, August 1974, Georg Thieme Stuttgart Avicenum Czechoslovak Medical Press Prague, 1975, S. 497; ; J. Investig. Dermatol. 82 (1984) 515; Biomedizinische Technik 31 (1986), 246; Z. Klin. Med. (Berlin DDR) 43 (1988), 185, 299, 945, 1093).

Eine Transillumination bzw. Transmission von Licht durch den Schädel eines Neugeborenen mit 7,5 cm Durchmesser wurde integral als "single frame image" wiedergegeben; außerdem erfolgte bei den Wellenlängen 840 nm und 760 nm die Transmission der Hand eines erwachsenen Menschen, wobei die Durchstrahlung integral für den Status mit und ohne Hypoxie dargestellt wurde und auch ein Blutgefäß sowie Knochen dabei unterschieden werden konnten. Die Autoren im u.g. Artikel kamen zu dem Schluß, daß das Gewebe bis zu mehreren Zentimetern Dicke für NIR- Strahlung suffizient transparent ist und eine Detektion transmittierter Photonen erlaubt, daß aber die räumliche Spezifik schlecht sei, obwohl die Auflösung der Strukturen bis zu einer Tiefe von letztlich 1 cm möglich ist ( Information processing in medical imaging. Proc. 9th conference, Washington D. C., 10.-14.06.1985. Martinus Nijhoff Publish. Portrecht 1986, S. 155)

Diesen bekannten fotoplethysmografischen bzw. Transilluminationsmethoden ist gemeinsam das integrale Messen von Volumina. Eine solche integrale Messung hat offensichtlich den Nachteil, nicht näher definierte Volumina zu messen.

Zur Messung von Mikrovolumina für medizinische Untersuchungen sind geeignete Meßsonden bekannt, die aus Lichtsender und Empfänger bestehen, bei denen Sender und Empfänger in einer Ebene und unmittelbar benachbart angeordnet sind, das Licht über Lichtleiter an den Meßort übertragen wird, wobei die Meßsonde als Nadel oder Hohlnadel gestaltet ist und infolge eines Durchmessers an der Spitze von 2 bis 20 $\mu$m auch für die Erfassung von mikrostrukturierten Volumenteilchen geeignet ist (DE- OS 3 009 901).

Ein Nachteil dieser Vorrichtung zur Messung von Mikrovolumenteilchen ist die invasive bzw. traumatische bzw. zerstörerische Zuführung der Meßsonde vor den Ort der zu messenden Mikrostruktur in Form einer Hohlnadel bzw. Mikropipette. Wenn mit dieser Hohlnadel, um an den Ort der Messung, beispielsweise des Gehirns, zu gelangen, Kopfhaut, Schädeldecke sowie Hirnhäute verletzt werden müssen, um danach bedingt atraumatisch das Gehirn zu messen, so geschieht dies keineswegs rückwirkungsfrei.

Mit der bekannten Vorrichtung werden in die zu untersuchende Probe Licht emittierend und elektrisch leitende Substanzen unter einem bestimmten Druck injiziert. Für das Gehirn bedeutet das über Injektionsnadeln oder Mikropipetten mit dieser Vorrichtung erfolgende Heranführen chemischer Substanzen einen Eingriff in die Funktionsweise dieses Organs mit Auslösung unwägbarer Reaktionen. Aber auch für die Beurteilung der Blutmikrozirkulation, die bekanntermaßen in lokalen diskreten Mikroarealen wesentlich geregelt wird (Europ. Neurol. 20 (1981), 200), stellt dieses Vorgehen einen möglichen Mangel dar, der durch die Messung entsteht und diese verfälscht. Bekannt ist die Laser- Scanning- Mikroskopie (Chip Nr. 1 Jan. (1989), 20, die eine zerstörungsfreie, rückwirkungsfreie Messung von Mikrostrukturen erlaubt. Bei der Laser- Mikroskopie ermöglicht eine punktförmige Lichtquelle über ein konfokales System, Oberflächen, Gewebe, Zellen und Mikrostrukturen innerhalb der Zelle auch an lebenden Präparaten dreidimensional darzustellen und zu vermessen, beispieslweise Chromosomen einer Küchenzwiebelwurzel in 16 verschiedenen Tiefen. Die optischen Schnitte gelangen im Abstand in 0,5 Mikrometern in durchgängiger Tiefenschärfe. Wesentlichen Anteil hat dabei die dreidimensionale Rekonstruktion der einzelnen Aufnahmepunkte. Das Betrachten technischer Materialien auch unterhalb der Oberfläche, z.B. die Ausrichtung von Lackpartikeln in Metallic- Lacken sowie die Beurteilung von Kriterien wie Porosität, Bruch- und Reißfestigkeit sind möglich. Sollen in der Tiefe bzw. in situ und in vivo relativ großer und inhomogener Makrostrukturen mit relativ geringen Strahlungsintensitäten im Milliwattbereich pro Quadratzentimeter Steradiant atraumatisch und rückwirkungsfrei schnelle räumliche und zeitliche Änderungen in Mikrostrukturen beliebiger Verteilung innerhalb dieser Makrostruktur in Echtzeit mehrdimensional dynamisch gemessen bzw. dargestellt werden, so ist durch die Laser- Mikroskopie dies nicht zu realisieren. Teilweise kann die Aufgabe, nämlich Mikrostrukturen beliebiger Verteilung in einer Makrostruktur mehrdimensional zu messen, durch bekannte magnetresonanz-tomografische Verfahren gelöst werden. Jedoch werden dazu Magnetfelder von mehreren Tesla benötigt, die nicht rückwirkungsfrei auf biologische Strukturen einwirken. Außerdem sind fotospektrometrische, die Lichtreflexion nutzende Vorrichtungen bzw. eine entsprechende Anordnung für eine noninvasive kontinuierliche atraumatische Anwendung in vivo zur Diganostik des Metabolismus in Körperorganen wie Gehirn und Herz bekannt (US 4 223 680; 4 281 645; 4 321 930; 4 380 240). Diese Vorrichtungen applizieren Licht mit zwei definierten Wellenlängen in das Gewebe, z.B. in den Wellenlängenbereichen von 700 bis 1300 nm ( im nahen Infrarot (NIR)- Bereich). Dabei wird mit Hilfe lichtempfindlicher Detektoren in einer verhältnismäßig großen Entfernung von einigen Zentimetern vom Eintragungsort die Reflexion bzw. Transmission gemessen. Dadurch kommt es zu keiner differentiellen, sondern zu einer integralen räumlichen Messung und Auswertung.

Diese hat den Nachteil, daß z.B. nur das gesamte Organ oder große Teile des Organs gemessen bzw. dargestellt werden können, also relativ große Volumina. Für die feinauflösende Tomografie und mehrdimensionale Darstellung ist ein derartig großes Volumenelement nicht zu gebrauchen; das gilt besonders, wenn mit Rückstreulicht gearbeitet werden soll.

Weiterhin ist bekannt, z.B. zur Erkennung des Brustkrebses, die Brust mit NIR- Strahlung zu durchstrahlen, aber auch hier wird nur die Transmissionsstrahlung gemessen und keine punktuell- differentielle Messung vorgenommen (DE 31 03 609). Bekannt ist auch ein Verfahren zur Beurteilung eines oder mehrerer Gewebeteilchen im Gehirn oder der weiblichen Brust, wobei die bekannten Algorithmen zur Computertomografie genutzt werden und das Durchstrahlungsprinzip angewendet wird (US 4 515 165, , DD 210 202, US-PS 4 570 638, DE 30 19 234 C2). Dabei werden Strahlen ($\lambda$ = 700 - 1300 nm) durch das Gewebe geschickt, die auf ihrem Wege abgeschwächt werdend Diese abgeschwächten Strahlen sind in bezug auf ihre Intensität nicht korrigierbar.

Konkret ist aus der DE 30 19 234 C2 eine Einrichtung zur Messung des Stoffwechsels eines Organs, z.B. des Herzens oder des Hirns bekannt, welche von einer Lichtquelle ausgeht, die im nahen Infrarotbereich zwischen 700 und 1300 nm Strahlung mindestens einer Meßwellenlänge sowie einer Bezugswellenlänge liefert. Diese Strahlung durchdringt das zu untersuchende Organ entweder in Durchleuchtungsrichtung oder unter Nutzung von Rückstreustrahlung. Die Meßwellenlänge wird so ausgewählt, daß diese im Bereich des Absorptionsbandes für spezifische Stoffwechselzustände liegt, vorzugsweise im Bereich des sauerstoffgesättigten und des sauerstoffverarmten Hämoglobins. Darüber hinaus sind Halterungen vorgesehen, mit deren Hilfe die Lichtstrahlung an einem bestimmten Punkt in den Körper eintreten kann, wobei am Austrittspunkt Lichtsensoren angeordnet werden, mit denen die Meß- und Bezugswellenlängen getrennt erfaßbar sind. Die Ausgangssignale der Lichtsensoren werden gemäß dortiger Lösung an eine Verarbeitungsschaltung gegeben, welche das unterschiedliche Absorptionsverhalten als Funktion der unterschiedlichen Wellenlängen bestimmt. Auch ist es aus der DE 30 19 234 C2 bekannt, eine Vielzahl von Lichtstrahlen in zwei- oder dreidimensionaler Verteilung mit unterschiedlichen Winkeln auf das zu untersuchende Organ zu richten, wobei den einzelnen Lichtstrahlen eine Vielzahl von entsprechenden Lichtsensoren bzw. -empfängern zugeordnet sind. Die einzelnen Lichtstrahlen werden in einer vorbestimmten Folge nacheinander ausgelöst und mit den Lichtsensoren bzw. -empfängern abgetastet, um durch aufeinanderfolgenden Vergleich der Meßwellenlängen mit den Bezugswellenlängen die Stoffwechselaktivität des untersuchten Organs in bestimmten räumlichen Bereichen zu ermitteln. Primär offenbart die genannte Lehre die Untersuchung des oxidativen Stoffwechsels im Bereich bestimmter Organe, wobei die erhaltene örtliche Auflösung relativ gering ist.

Auch ist ein Verfahren und eine Vorrichtung (DE-OS 3 724 593) bekannt, um das von einer untersuchten Probenflüssigkeit unterschiedlich absorbierte Licht auf einem vorbestimmten Betrag zu halten, was durch Einstellen der Länge des Lichtweges in der Probe geschieht. Mit der Anwendung dieses Verfahrens bzw. der Vorrichtung, die die Veränderung der Weglänge sowie eine Homogenität der zu untersuchenden Probe zur Voraussetzung hat, läßt sich die Struktur eines Volumenflusses nicht dynamisch bestimmen, wenn in einer inhomogenen Struktur diese sich räumlich und phasisch im Millisekundenbereich ändert.

Zur Ermittlung der Blutflußgeschwindigkeit in großen und kleinen Gefäßen ist die Doppler- Flow-Methode bekannt (Bibliotheca. anat. No. 18, Krager, Basel 1979, S. 16; Terapiewoche 32 (1982), 5082; Int. J. Microzirkulation: Clin. exp. 5 (1986), 73; Investig.Dermatol. 82 (1984), 515). Mit dieser Methode ist es möglich, den Blutkörperchenfluß in seiner Geschwindigkeit (velocity) in den Mikrogefäßen zu messen. Nachteilig hierbei ist, daß untrennbar auch die langsamen Flußgeschwindigkeiten in den Mikrogefäßen mitgemessen werden. Auch wird durch die Doppler- Flow- Methodik nur die Fließgeschwindigkeit (velocity) der Blutbestandteile in den Gefäßen bestimmt; der tatsächliche Volumenfluß der roten Blutzellen, d.h. die Menge der roten Blutzellen, die einen bestimmten Querschnitt in einer bestimmten Zeit durchsetzt - dieser Volumenfluß, der bei einem enggestellten Mikrogefäß relativ zur Flußgeschwindigkeit gering ist, der bei einem weitgestellten Mikrogefäß aber relativ zur Flußgeschwindigkeit groß ist-, kann mit der Doppler-Flow- Methode nicht bestimmt werden. Das heißt, die Fließgegeschwindigkeiten der roten Blutzellen im Gefäßsysteme ist abzugrenzen vom Volumenfluß durch das Gefäßsystem. Dieser Volumenfluß betrifft sowohl zelluläre als auch nichtzelluläre Bestandteile des Blutes. Im Makrogefäßsystem ist der Volumenfluß unter Einrechnung des Hagen- Poiseuille'schen Gesetzes mit Flow- Metern meßbar. Im Mikrozirkulationsbereich gehorchen die einzelnen Parameter nicht mehr dem angegebenen Gesetz, sondern zeigen Abweichungen (Dtsch. Arch. f. Klin. Medizin 169 (1930),212; Klin. Wschr. 7 (1928) 100). Bekannt sind außerdem Methoden zur Ermittlung des Blutflusses (blood flow), der über die Gesamtflußgröße des Volumenflusses, d.h. von zellulären und nicht zellulären Bestandteilen des Blutes Auskunft gibt. Dabei wird nicht differenziert zwischen der Messung des Volumenflusses der roten Blutzellen und des Volumenflusses des nichtzellulären Blutplasmas. Der Blutfluß wird in ml pro 100 g Gewebe pro Minute angegeben und nichtselektiv im Gesamtgefäßsystem, z.B. mit der Xenon- 133-Isotopenmethode bestimmt ( J. Neurol. Neursurg. Psychiat. 35 1972), 285; L' Encéphále 4 (1978), 233; Brain 94 (1971), 635).

Bei einem neueren computertomografischen Verfahren zur Messung der Hirndurchblutung, der "Xenon- Computer- Tomografie" wird nichtradioaktives Xenon inhaliert. Dieses gelangt über du Blutbahn zu allen Organen, auch zum Gehirns Die Geschwindigkeit, mit der sich die Anreicherung von Xenon im Gewebe vollzieht, ist ein Maß für die Durchblutung (Chip Nr. 8 - August 1988, S. 241...244). Nachteilig ist offensichtlich die hierbei zusätzliche Inhalierung eines nichtradioaktiven Gases.

Der Erfindung liegt daher die Aufgabe zugrunde, ein Verfahren und eine Anordnung zur Untersuchung von Strukturen unter Ausnutzung einer gezielten örtlich und volumendefinierten Streulichtmessung und der Verwendung von matrixförmigen angeordneten IR- Lichtquellen und IR- Empfängern anzugeben, wobei auch sensorferne Strukturen erfaßt werden sollen, eine feinauflösende Darstellung möglich ist und die Strukturen punktuell dynamisch als Volumenfluß bestimmt und dargestellt werden können sowie der Volumenfluß selektiv in den kapillaren Gefäßen und bei beliebiger Lokalisation innerhalb inhomogener, räumlich, zeitlich und qualitativ in ihrer stofflichen Zuammensetzung sich ändernder Strukturen sowie mehrdimensional in Echtzeit bestimmt werden können. Die Lösung der Aufgabe erfolgt mit einem Verfahren nach Anspruch 1 sowie einer Anordnung nach Anspruch 10.

Erfindungsgemäß werden in einer Ebene angeordnete Lichtquellen und Empfänger als Flächenstrahler in z- Richtung zum messenden bzw. darzustellenden Objekt geführt, wobei die Lichtquellen eine IR- Strahlung im NIR- Bereich mit einer bestimmten Strahlungsintensität aussenden, und wobei die IR- emittierenden Dioden mit einem Spitzenstrom um 1 A bei einer Impulsfrequenz um 100 kHz angesteuert werden und wobei nach Empfang des Lichtes eine Separierung des Signals in Sinne einer Echtzeitspektralanalyse der Signale erfolgt. Anschließend werden die für bestimmte Frequenzbänder integral und differentiell gewonnenen Signale entsprechenden Farben zugeordnet und auf dem Monitor als Funktion der Intensität, des Ortes, der Zeit und der Wellenlänge dargestellte Die Echtzeitspektralanalyse wird vorzugsweise für die Frequenzbänder 0,1 - 4 Hz, 4 - 8 Hz mit wählbarer Verschiebung bis zu 1 kHz durchgeführt.

Der Einsatz des Flächenstrahlers erfolgt in x-y-z- Richtung im mehrdimensionalen Multiplexbetrieb dergestalt, daß mit dem Flächenstrahler sich nahezu überlappende oder auch nicht überlappende Flächen des darzustellenden Objektes bestrahlt werden. Diese Flächenbestrahlung führt zu einer axialen Intensitätsfokussierung im bestrahlten lichtstreuenden Objekt und ermöglicht eine selektive Illuminierung des jeweils senderfernsten Volumenelementes im darzustellenden, in sich nicht homogenen und sich räumlich, zeitlich und qualitativ in seiner stofflichen Zusammensetzung ändernden Objekt. Dieses senderfernste selektiv illuminierte Volumenelement wird mit Strahlintensitäten vorzugsweise in Größen bis zu 100 mW . cm$^{-2}$.sr$^{-1}$ des Flächenstrahlers erreicht, indem durch Multiplexbetrieb in z-Richtung das jeweils senderfernste Volumenelement intensitätsgestuft und intensitätsproportional in zunehmend tiefere bzw. senderfernere Schichten verlagert und dort durch mehrdimensionalen Multiplexbetrieb differentiell erfaßt sowie als Rückstreulicht detektiert wird. Die Erfassung von Volumenelementen kann auch durch Detektion des transmittierten Lichtes erfolgen bzw. sowohl rückgestreutes als auch transmittiertes Licht werden zur Darstellung der Strukturen verwendete Die Bestrahlung des darzustellenden Objektes wird im Pulsbetrieb realisiert, wobei infolge der axial erfolgenden Intensitätsfokussierung durch Vergrößerung der mit einer bestimmten Intensität pro Quadratzentimeter bestrahlten Fläche eine zunehmende Eindringtiefe axial durch die Flächenbestrahlung erreicht wird, was eine axiale Detektion des rückgestreuten und/ oder transmittierten Lichtes ermöglicht

Der Flächenstrahlungs-Empfangssensor besteht aus einzelnen Bauelementen, mit denen die darzustellende Struktur bzw. das Objekt netzartig teilweise oder ganz überzogen wird, wobei die einzelnen Bauelemente in einer Makro- Sende- Empfangs-Matrix angeordnet sind und an der unregelmäßigen Oberfläche der darzustellenden Struktur in verschiedenen Winkeln zueinander mehr oder weniger anliegen, was bei einer das einzelne Bauelemente übergreifenden großflächigen Bestrahlung zu verschiedenen Strahlrichtungen der einzelnen Bauelemente führt. Aus diesem Grunde werden auf jedem Bauelement an der dem dazustellenden Objekt abgewandten Seite ein oder mehrere Strahler angeordnet, die in Minus- z- Richtung strahlen und die der Positionierung sowohl des vom Flächenstrahler-Empfänger überzogenen Objektes als auch zur Bestimmung der Position der einzelnen Bauelemente auf dem Objekt, insbesondere deren Winkelstellung zueinander, in einem Raumkoordinatensystem dienen. Da der Flächenstrahler wahlweise in kleinschrittigem Multiplexbetrieb in x-y- Richtung angesteuert wird, um so sich nahezu überlappende Flächen zu bestrahlen bzw. sich nahezu überlappende Volumenelemente in der Tiefe des darzustellenden Objektes selektiv zu illuminieren und diese Volumenelemente axial ohne Überlappung detektieren zu können, sind die einzelnen Bauelemente mikrostrukturiert. Dazu werden Sender und Empfänger in einem Abstand von 10 - 20 μm als Mikro-Matrix auf diesem Bauelement sowie als Strahler verschiedener Wellenlängen um den Empfänger angeordnet. Dies erlaubt die IR-spektroskopische Darstellung ausgewählter Stoffe im NIR- Bereich bzw. im Oberschwingungsbereich fundamentaler Molekülschwingungen mit einer Auflösung im Molekülgrößen-bzw. Mikrometerbereich, wobei die detektierten Stoffe verschiedenen Farben zugeordnet und auf dem Monitor als Funktion der Intensität, des Ortes und der Zeit, beispielsweise zeitspektralanalytisch dargestellt werden.

Mit dem mikro- und makrostrukturierten Flächensensor werden IR-spektroskopische Darstellungen von Volumenelementen in situ bzw. innerhalb inhomogener, sich zeitlich, räumlich und qualitativ in ihrer stofflichen Zusammensetzung ändernder Volumenelemente gleicher Abmaße mit identischer Intensität illuminiert, wobei die Steuerung der Intensität zum Erreichen dieser Illumination dynamisch durch punktuell- differentiell gemessene volumenelement- spezifische Schwächungskoeffizienten in Echtzeit erfolgt und diese von ein und dem selben Volumenelement erhaltenen, sich zeitlich qualitativ unterscheidenden Schwächungsgradienten bzw. volumenelement-spezifischen Schwächungsgradienten- Profile im Rechner gespeichert werden und zur exakten räumlichen Zuordnung der mehrfach gemessenen Volumenelemente in der darzustellenden Makrostruktur genutzt werden.

Es werden zur Untersuchung und Darstellung sowohl die mit dem Flächenstrahler-Empfänger integral erhaltenen als auch differentiell gewonnenen Signale einschließlich einer Kontrastdarstellung von Strukturen innerhalb der dargestellten Makrostruktur genutzt.

Bestimmte in Echtzeit punktuell- differentiell gemessene volumenelement- spezifische Schwächungskoeffizienten werden zur selektiven Bestimmung und Darstellung des Volumenflusses einer Suspension in einem kapillaren bzw. Mikrogefäßsystem innerhalb eines aus großen und kleinen Gefäßen bestehenden Gesamtgefäßsystems herangezogen. Damit werden Anteile bzw. flüssige und feste Bestandteile der Suspension bestimmt, die pro Volumen-und Zeiteinheit als integrale Lichtabsorptionsgrößen bzw. als Differenz sich phasisch und lichtabsorptions- proportional ändernde Volumenflußgrößen das Kapillarsystem durchsetzen. Diese Differenz sich zeitphasisch und lichtabsorptions-proportional ändernder Partikelvolumenflußgrößen wird als Differenz des systolisch proportionalen zum diastolisch proportionalen Volumenfluß der roten Blutzellen bzw. als Differenz der Quotienten

$$\frac{\text{Zellvolumenfluß}_{systol.}}{\text{Plasmavolumenfluß}_{systol.}} - \frac{\text{Zellvolumenfluß}_{diastol.}}{\text{Plasmavolumenfluß}_{diastol.}} = \zeta$$

bestimmt, wobei nach Empfang des Lichtes das detektierte Signal einer Echtzeitspektralanalyse unterzogen wird und die Bestimmung bzw. Darstellung auf einem Monitor als Funktion der Intensität, des Ortes, der Zeit und der Wellenlänge erfolgt.

Mit dieser Beziehung können die partikulären und nichtpartikulären Anteile einer Suspension beispielsweise des Blutes sowohl in biologischen bzw. natürlich entstandenen als auch in künstlichen bzw. technisch gefertigten Gesamtgefäßsystemen und Filtern selektiv für das jeweilige kapillare System bestimmt werden. Für bestimmte Amplituden und die Zeitpunkte $t_1$ und $t_2$ kann die Differenz der sich mit der Herzaktion bzw. systolisch/ diastolisch lichtabsortionsproportional ändernden Partikelvolumenflußgrößen des AC- und DC- Kanals als Differenzquotient

$$\frac{A_{ac_1} - A_{ac_2}}{A_{dc_1} - A_{dc_2}}$$

bestimmt werden. Diese Beziehung läßt sich zur Bestimmung des Selbstregulationsbereiches des Mikrozirkulationssystems verwenden.

Der Vorteil der Erfindung ist es, ein Verfahren und ein Anordnung zu erhalten, durch die mit robusten und relativ preiswerten optoelektronischen Bauelementen sowie durch eine problemlose Objekt- Applikation, die beispielsweise komplizierte, anfällige und aufwendige Laser- Vorrichtungen überflüssig macht, mit relativ geringen Lichtintensitäten von etwa 10 mW pro Quadratzentimeter Steradiant kleine und große inhomogene Strukturen unegaler Oberfläche bestrahlt werden können, um deren innere Strukturen statisch und dynamisch, beispielsweise den Volumenfluß in diesen Strukturen sensorfern in situ und/ oder in vivo mehrdimensional, zerstörungs- und weitgehend rückwirkungsfrei in Echtzeit bestimmen und feinaufgelöst darstellem zu können.

Die Erfindung soll nachfolgend anhand eines Ausführungsbeispieles unter Bezugnahme auf die zugehörigen Zeichnungen näher erläutert werden:

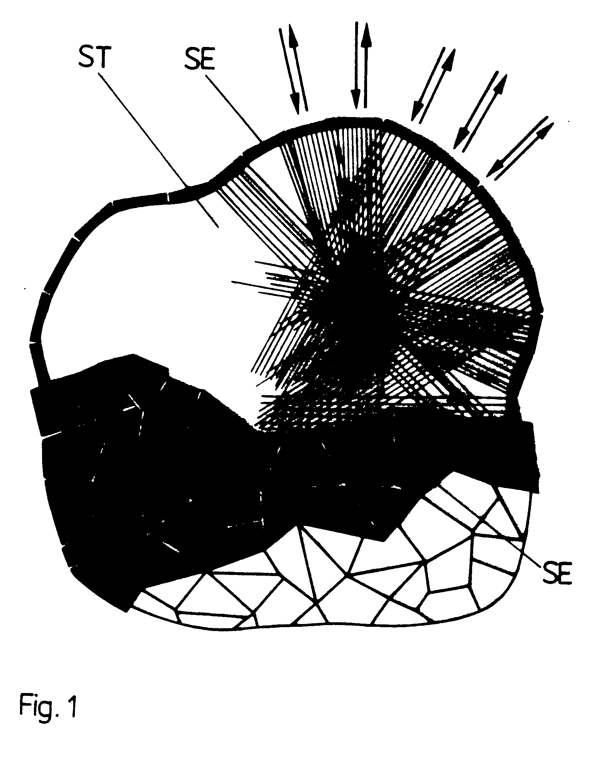

Fig. 1    zeigt schematische ein zu untersuchendes und darzustellendes Objekt als Schnittebene durch den oberen Teil sowie dessen unregelmäßige Oberfläche im unteren Teil des Objektes. Die Oberfläche ist von IR-Flächen-Sende- und Empfangs- Bauelementen netzartig in Form einer Matrix überzogen. Durch die Mikrostruktur dieser Bauelemente wird eine bauelementeübergreifende Flächenstrahlung und axiale Detektion der rückgestreuten IR- Strahlung im mehrdimensionalen Multiplexbetrieb in x-y- Richtung ermöglicht.

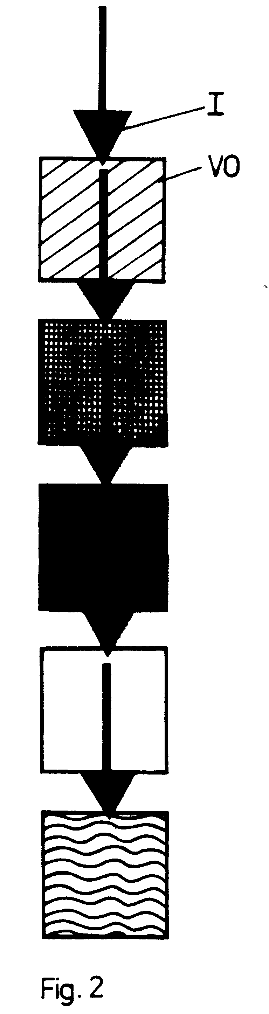

Fig. 2     zeigt schematisch die sequentielle selektive Illuminierung von unterschiedlich quantitativ und qualitativ lichtschwächenden Mikrostrukturen bzw. Volumenelementen VO gleicher Abmaße in z- Richtung eines x-y-z- Koordinatensystems mit der Illuminationsintensität I in einer inhomogenen durch die Gesamtheit der Volumenelemente gebildeten Makrostruktur ST.

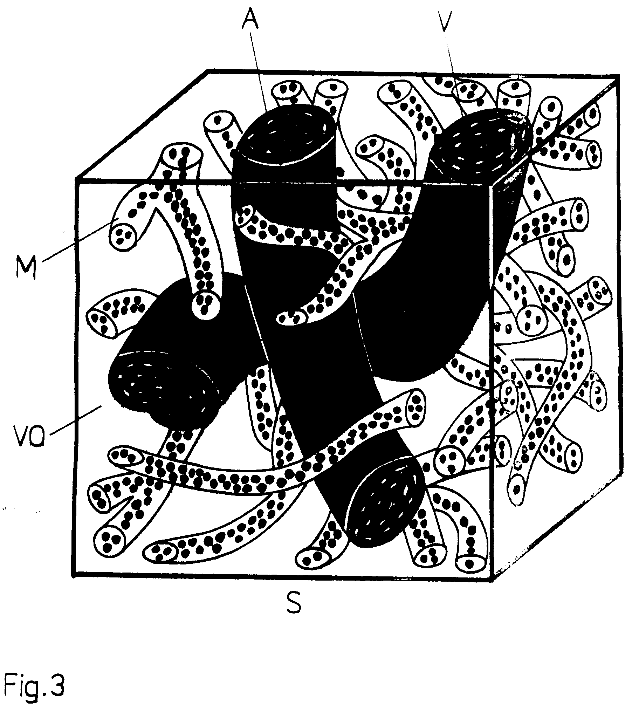

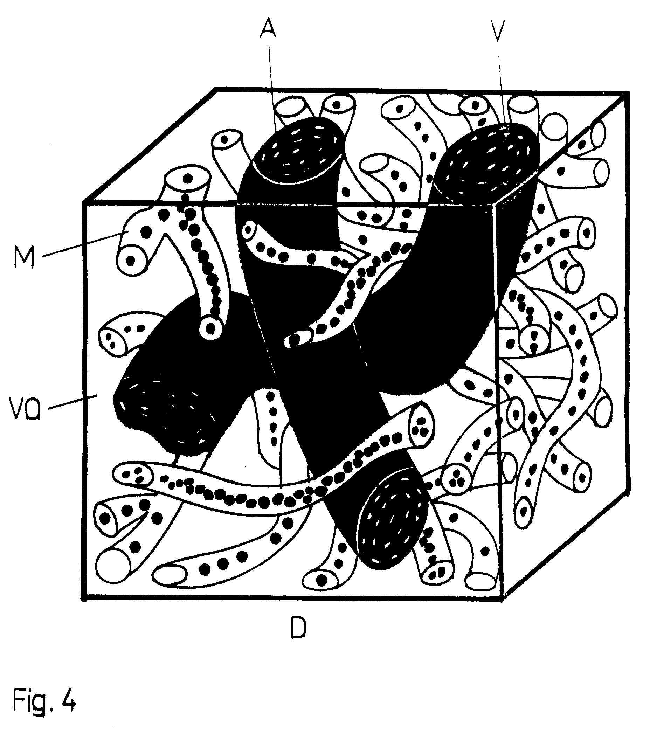

Fig. 3,4     zeigen ein und dasselbe Volumenelement VO in der systolischen Phase S und diastolischen Phase D der Herzaktion mit zeitphasischen unverändertem Hämatokrit in den Makrogefäßen A, V und zeitphasisch sich änderndem Hämatokrit in den Kapillaren bzw. Mikrogefäßen M, in denen die roten Blutzellen axial strömen und das Blutplasma randständig strömt. In der systolischen Phase weisen die Mikrogefäße einen höheren Anteil an roten Blutzellen auf als in der diastolischen Phase. Die Menge der Erythrozyten in den Kapillaren ist dem Volumenfluß der roten Blutzellen proportional.

Die Erfindung soll am Beispiel des Gehirns erläutert werden, ist aber keinesfalls auf dieses Organ oder auf biologisches Gewebe beschränkt.

In Figur 1 und 2 ist die Verfahrensweise dargelegt, mit der ein inhomogenes, relativ großes, darzustellendes Objekt mittels eines Flächenstrahlers, dessen Strahlungsquerschnitt der Fläche des darzustellenden Objektes entsprechen kann, bestrahlt wird. Die Pfeilpaare repräsentieren hierbei Sektionen auf der dargestellten Schnittebene, beispielsweise des Hirnschädels, über die der Flächenstrahler in jeweils hier eingezeichneten kleinen Multiplexschritten in x-y-Richtung geführt wird: Bei jedem Multiplexschritt wird axial der Rückstreustrahl gemessen. Diese Messung erfolgt, da auch in z-Richtung im Multiplexbetrieb intensitätsgestuft bestrahlt wird, mehrdimensional differentiell vom jeweils senderfernsten Volumenelement, da dieses infolge der axialen Intensitätsfokussierung durch die Flächenbestrahlung intensitätsproportional selektiv illuminiert wird. Durch Echtzeitermittlung der volumenelementespezifischen NIR-Schwächungsgradienten- Profile, die dem einzelnen Volumenelement sozusagen ein unverwechselbares Profil geben, wird eine exakte räumliche Zuordnung der Volumenelemente, die mehrfach innerhalb der Makrostruktur beispielsweise des Gehirns gemessen wurden, ermöglicht. Durch Anordnung von Strahlern auf der dem darzustellenden Objekt Gehirn abgewandten Seite der Bauelemente und deren Strahlen in Minus-z-Richtung, wird sowohl die Position des darzustellenden Objektes Gehirn bzw. Gehirnschädel als auch die Winkelstellung der auf dieser unregelmäßigen Schädeloberfläche angeordneten Bauelemente zueinander bestimmbar, so daß durch die zusätzliche Anordnung ein weiterer Faktor für eine exakt räumliche Zuordnung der axial gemessenen Volumenelemente der darzustellenden Struktur, auch in deren Tiefe, gegeben ist.

Da das darzustellende Objekt Gehirn IR- spektroskopisch gemessen und dargestellt werden soll, ist eine für jedes Volumenelement identische Illuminations- Intensität I zu gewährleisten, wenn die einzelnen Volumenelemente miteinander verglichen werden bzw. in ihrem Intensitäts- bzw. Grauwertabstufungen NIR- tomografisch dargestellt werden sollen. Dies erfolgt, indem die echtzeitermittelten NIR- Schwächungskoeffizienten von jedem gemessenen Volumenelement bei der Ansteuerung des nächstferneren, selektiv zu illuminierenden Volumenelementes berücksichtigt werden.

Fig. 3 und 4 veranschaulichen, wie innerhalb einer inhomogenen Struktur Volumenelemente gleicher Abmaße, bei vorausgesetzter Illuminierung dieser Volumenelemente mit einer für alle gemessenen Volumenelemente identischen Intensität, phasische Änderungen aufweisen, die für die selektive Bestimmung des Volumenflusses durch das Mikrozirkulationssystem dieser Struktur genutzt werden können. In einem sowohl Makrogefäße als auch kapillare Mikrogefäße umfassenden Gesamtgefäßsystem separieren sich und strömen aufgrund des bekannten physikalischen Fahraeus-Effektes die Partikelchen einer Suspension axial (axial proportional zur Partikelgröße).

In der systolischen Phase der Herzaktion ist dieser Volumenfluß gegenüber der diastolischen Phase erhöht. Die durch den Fahraeus- Effekt erzwungene Separierung und axiale Strömung der partikulären Blutbestandteile im kapillaren Gefäßsystem ist als Durchsatz der roten Blutzellen durch das Mikrogefäßsystem proportional der Strömungsgeschwindigkeit der roten Blutzellen im Mikrogefäßsystem zu verstehen. Wird die Lichtabsorption der roten Blutzellen gemessen, so läßt sich beispielsweise bei Hämoglobin/ Oxihämoglobin- isobestischen Wellenlängen diese Absorption als systolisch/diastolische Differenz des Volumenflusses roter Blutzellen selektiv und quantitativ, d.h. der zeitphasischen Lichtabsorptionsdifferenz der roten Blutzellen proportional, im Mikrozirkulationssystem innerhalb des Gesamtgefäßsystems volumenproportional zur Menge der roten Blutzellen, die ein Volumenelement pro Zeiteinheit durchsetzen, bestimmen. Dies kann selektiv für verschiedene Schichten eines Gewebes, beispielsweise des Herzens oder des Gehirns oder auch für wählbare Regionen und Schichten in technisch gefertigten Gefäßsystemen und Filtern, denen Gefäße und lichtstreuende und absorbierende Medien eingebettet sind, erfolgen.

Zum Beispiel als Mapping von 16 Regionen des Kopfes, wobei die Meßstellen in Anlehnung an das für die klinische Elektroenzephalografie international gebräuchliche Ten- Twenty-System gewählt werden, da von den gleichen Ableitpunkten simultan zum mikrozirkulatorischen Volumenfluß des Gehirns auch ein event- related bzw. ein ereignisbezogenes Mapping vom EEG auf dem Monitor wiedergegeben werden kann; auch die Interaktion von mikrozirkulatorischem Volumenfluß und EEG läßt sich als Mapping darstellen, da beide, EEG und Gehirn- Mikrozirkulation, zeitspektral analysiert werden können.

Im Gegensatz zum EEG werden Schichttiefen des Gehirns selektiv in ihrem mikrozirkulatorischen Volumenfluß bestimmt. Durch eine Simultanregistrierung von mikrozirkulatorischem Volumenfluß und EEG läßt sich die mit dem EEG nur grob mögliche Ortsauflösung, beispielsweise eines Ereignisses "event-related" feiner örtlich auflösen bzw. relativ präzise die Schichttiefen des Gehirns an 16 verschiedenen Regionen des Kopfes in 16 Intensitäts- bzw. Graustufen für eine gewählte Einzelfrequenz der Powerspektren oder für mehrere Frequenzbänder, die den Farben Rot, Grün, Blau auf dem Monitor zugeordnet werden und als Mapping separat für jedes Frequenzband für einen bestimmten Zeitabschnitt darstellen.

Es werden so dynamische Vorgänge des Volumenflusses als Funktion der Intensität, des Ortes, der Zeit und der Wellenlänge bestimmbar. Bei Anwendung einer feinauflösenden Sensor- Matrix lassen sich dynamische NIR- Tomogramme realisieren.

## Patentansprüche

1.  Verfahren zur Untersuchung von biologischen und technischen Volumenstrukturen unter Nutzung örtlich und volumendefinierter Streulichtmessung mit einer Vielzahl von flächig angeordneten IR-Lichtquellen und -empfängern, welche einen mikrostrukturierten Flächenstrahler bilden,
    mit folgenden Schritten:

    -   Anordnen des Flächenstrahlers auf einem Abschnitt der Oberfläche der zu untersuchenden Volumenstruktur, wobei die Strahlung des Flächenstrahlers in z-Richtung emittiert wird und in die Volumenstruktur eintritt;

    -   Beaufschlagen der IR-Lichtquellen mit Energie derart, daß ein Pulsbetrieb mit vorgegebener Impulsfolgefrequenz von IR-Dioden, die als Lichtquellen verwendet werden, erfolgt;

    -   schrittweises Bewegen des Flächenstrahlers über die Oberfläche der Volumenstruktur und Messen der räumlich zugeordneten Rückstreustrahlung mehrfach am selben Ort, jedoch bei unterschiedlicher Sendeintensität zur Erfassung unterschiedlicher Volumenabschnitte der Struktur und zur Ermittlung eines Schwächungskoeffizienten;

    -   Speichern der erhaltenen Schwächungskoeffizienten und Bestimmung eines Schwächungskoeffizienten-Profils der Volumenelemente durch Mehrfach- oder Wiederholungsmessung bei unterschiedlichen Einstrahlungswinkeln und/oder unterschiedlicher Wellenlänge und/oder zu unterschiedlichen Zeitpunkten sowie dreidimensionale Darstellung der erhaltenen Schwächungskoeffizienten-Profile mittels eines an sich bekannten Bildverarbeitungssystems.

2.  Verfahren nach Anspruch 1,
    dadurch gekennzeichnet,
    daß die IR-Strahlung axial intensitätsfokussiert wird und der Intensitätsfokus des Flächenstrahlers ein Drittel des Durchmessers eines als Ring strahlenden Flächenstrahlers beträgt.

3.  Verfahren nach Anspruch 1,
    dadurch gekennzeichnet,
    daß die Strahlungsintensität der als Flächenstrahler angeordneten Lichtquellen in Größen bis 100 mW $\cdot$ cm$^{-2}$ $\cdot$ sr$^{-1}$ geändert wird.

4.  Verfahren nach Anspruch 1,
    dadurch gekennzeichnet,
    daß die Ansteuerung der IR-Lichtquellen im zeitlichen, räumlichen und Wellenlängen-Multiplex erfolgt und die Volumenelemente zu den nächstbenachbarten mehrdimensional differentiell in X-Y- und Z-Richtung gemessen werden.

5.  Verfahren nach Anspruch 1,
    dadurch gekennzeichnet,
    daß der Empfang des transmittierten Streulichtes axial erfolgt.

6.  Verfahren nach Anspruch 1,
    dadurch gekennzeichnet,

daß die IR-Spektren der im inhomogenen Objekt vorhandenen molekularen Stoffe in deren Oberschwingungsbereich gemessen und aufgelöst und im NIR-Bereich dargestellt werden.

7.  Verfahren nach Anspruch 1
    dadurch gekennzeichnet,
    daß zur selektiven Darstellung von Volumenelementen der Strahlungsquerschnitt geändert wird.

8.  Verfahren nach Anspruch 1,
    dadurch gekennzeichnet,
    daß zur Kontrastdarstellung bestimmter Strukturen in situ bestimmte volumenelement-spezifische Schwächungsgradienten benutzt werden.

9.  Verfahren nach Anspruch 1,
    dadurch gekennzeichnet,
    daß die Differenz zwischen sich zeitphasisch und lichtabsorptions-proportional anderenden Partikelvolumenflußgrößen funktionell als Differenz des systolisch proportionalen zum diastolisch proportionalen Volumenfluß der roten Blutzellen bzw. die Differenz der Quotienten

$$\frac{\text{Zellvolumenfluß}_{systol.}}{\text{Plasmavolumenfluß}_{systol.}} - \frac{\text{Zellvolumenfluß}_{diastol.}}{\text{Plasmavolumenfluß}_{diastol.}} = \zeta$$

bestimmt wird.

10. Anordnung zur Durchführung des Verfahrens nach einem der vorangegangenen Ansprüche unter Ausnutzung einer gezielten örtlich und volumendefinierten Streulichtmessung mit mehreren nebeneinander in einer X-Y-Ebene angeordneten IR-Lichtquellen und -empfängern, welche einen mikrostrukturierten Flächenstrahler bilden, mit :

    -   Mitteln zum Anordnen des Flächenstrahlers auf einem Abschnitt der Oberfläche der zu untersuchenden Volumenstruktur, wobei die Strahlung des Flächenstrahlers in z-Richtung emittiert wird und in die Volumenstruktur eintritt, sowie in entgegengesetzter Richtung ( -z- Richtung) austritt.

    -   Mitteln zum Beaufschlagen der IR-Lichtquellen mit Energie derart, daß ein Pulsbetrieb mit vorgegebener Impulsfolgefrequenz von IR-Dioden, die als Lichtquellen verwendet werden, erfolgt;

    -   Mitteln zum schrittweisen Bewegen des Flächenstrahlers über die Oberfläche der Volumenstruktur und Messen der räumlich zugeordneten Rückstreustrahlung mehrfach am selben Ort, jedoch bei unterschiedlicher Sendeintensität zur Erfassung unterschiedlicher Volumenabschnitte der Struktur und zur Ermittlung eines Schwächungskoeffizienten;

    -   Mitteln zum Speichern der erhaltenen Schwächungskoeffizienten und Bestimmung eines Schwächungskoeffizienten-Profils der Volumenelemente durch Mehrfach- oder Wiederholungsmessung bei unterschiedlichen Einstrahlungswinkeln und/oder unterschiedlicher Wellenlänge und/oder zu unterschiedlichen Zeitpunkten sowie dreidimensionale Darstellung der erhaltenen Schwächungskoeffizienten-Profile mittels eines an sich bekannten Bildverarbeitungssystems.

11. Anordnung nach Anspruch 10,
    dadurch gekennzeichnet,
    daß die einzelnen Sender- und Empfänger-Bauelemente der großflächigen Sender-Empfänger-Matrix einen Durchmesser im Millimeter- bis Zentimeterbereich aufweisen.

12. Anordnung nach Anspruch 10,
    dadurch gekennzeichnet,
    daß die Sender und Empfänger in den mikrostrukturierten Bauelementen in einem Abstand von 10 - 20 µm angeordnet sind.

13. Anordnung nach Anspruch 10,

EP 0 387 793 B1

dadurch gekennzeichnet,
daß zum Empfänger angeordnete Strahler verschiedener Wellenlängen die Mikrostrukturlemente des Bauelementes bilden.

## Claims

1. Method of investigating biological and industrial volume structures using local and volume-defined measurement or diffused light with a plurality of IR light sources and receivers arranged in a plane, which form a microstructured surface emitter, with the following steps:

   - locating the surface emitter on a section of the surface of the volume structure to be investigated, the radiation of the surface emitter being emitted in the z-direction and entering the volume structure;
   - charging the IR light sources with energy in such a way that pulsed operation results with a predetermined pulse sequence frequency of IR diodes, which are used as light sources;
   - staged movement of the surface emitter across the surface of the volume structure and measurement of the spatially associated backscatter radiation a plurality of times at the same place, yet with a different emission intensity in order to detect different volume sections of the structure and in order to determine an extinction coefficient;
   - storage of the extinction coefficients obtained and determination of an extinction coefficient profile of the volume elements by multiple or repeated measurements at different radiation angles and/or at a different wavelength and/or different points in time, and three-dimensional representation of the extinction coefficient profiles obtained by means of an image processing system known per se.

2. Method according to claim 1,
   characterised in that
   the IR radiation is axially intensity-focused, and the intensity focus of the surface emitter comes to a third of the diameter of a surface emitter radiating as a ring.

3. Method according to claim 1,
   characterised in that
   the radiation intensity of the light sources arranged as surface emitters is altered in magnitudes up to 100 mW $\cdot$ cm$^{-2}$ $\cdot$ sr$^{-1}$

4. Method according to claim 1,
   characterised in that
   the IR light sources are triggered in chronological, spatial and wavelength multiplex, and the volume elements are measured against the most adjacent one, differentially and multi-dimensionally in the X, Y and Z directions.

5. Method according to claim 1,
   characterized in that
   the transmitted diffused light is received axially.

6. Method according to claim 1,
   characterized in that
   the IR-spectra of the molecular materials present in the inhomogeneous object are measured in their harmonic range and resolved, and are represented in the NIR range.

7. Method according to claim 1,
   characterized in that
   the cross-section of the radiation is altered for selective representation of volume elements.

8. Method according to claim 1,
   characterized in that
   defined volume element-specific extinction gradients are used for contrasting representation of specific structures in situ.

9. Method according to claim 1,
   characterized in that

9

the difference between particle volume flow magnitudes altering in time phase and proportional to light absorption is determined functionally as the difference between the systolic proportional and the diastolic proportional volume flow of the red blood cells, or the difference between the quotients:

$$\frac{\text{Cell volume flow}_{\text{systol.}}}{\text{Plasma volume flow}_{\text{systol.}}} - \frac{\text{Cell volume flow}_{\text{diastol.}}}{\text{Plasma volume flow}_{\text{diastol.}}} =$$

**10.** Arrangement for carrying out the method according to one of the preceding claims, using a controlled local and volume-defined measurement of diffused light with a plurality of IR-light sources and receivers located next to one another in an X-Y plane, and which form a microstructured surface emitter, with:

- means for arranging the surface emitter on a section of the surface of the volume structure to be investigated, the radiation of the surface emitter being emitted in the Z direction and entering the volume structure, and emerging in the opposite direction (Z direction),

- means for charging the IR-light sources with energy in such a way that pulsed operation results with a predetermined pulse sequence frequency of IR diodes which are used as light sources,

- means for staged movement of the surface emitter across the surface of the volume structure and measurement of the spatially associated backscatter radiation a plurality of times at the same place, yet with a different transmission intensity for detecting different volume sections of the structure and in order to determine an extinction coefficient;

- means for storing the extinction coefficients obtained and determination of an extinction coefficient profile of the volume elements by multiple or repeated measurement at different radiation angles and/or at a different wavelength and/or different points in time, and three-dimensional representation of the extinction coefficient profiles obtained by means of an image processing system known per se.

**11.** Arrangement according to claim 10,
characterised in that
the individual transmitter and receiver components of the large-area transmitter-receiver matrix have a diameter in the millimetre to centimetre range.

**12.** Arrangement according to claim 10,
characterised in that
the transmitters and receivers are located in the microstructured components at a spacing of 10-20 ◊m.

**13.** Arrangement according to claim 10,
characterised in that
radiators of various wavelengths, located at the receiver, form the microstructure elements of the component.

**Revendications**

**1.** Procédé pour analyser des structures biologiques et techniques tridimensionnelles en utilisant la mesure d'une lumière diffusée définie sur le plan local et en volume avec une multitude de sources et de récepteurs de lumière infrarouge agencés en surface, qui forment un dispositif rayonnant en surface micro-structuré, comprenant les étapes suivantes :

- on agence le dispositif rayonnant en surface sur un secteur de la surface de la structure tridimensionnelle à analyser, le rayonnement du dispositif rayonnant en surface étant émis en direction z et entrant dans la structure tridimensionnelle;
- on alimente en énergie les sources de lumière infrarouge, de telle sorte qu'il s'effectue un fonctionnement par impulsions avec une fréquence d'impulsions successives prédéterminée de diodes à infrarouge qui sont utilisées comme sources de lumière;
- on déplace pas à pas le dispositif rayonnant en surface sur la surface de la structure tridimensionnelle et on mesure le rayonnement rétrodiffusé associé dans l'espace plusieurs fois au même emplacement mais avec différentes intensités d'émission pour saisir différents secteurs de volume de la structure et pour détecter un coefficient d'affaiblissement;

- on mémorise les coefficients d'affaiblissement obtenus et on détermine un profil des coefficients d'affaiblissement des éléments de volume par une mesure multiple ou répétée sous différents angles d'incidence et/ou avec différentes longueurs d'onde et/ou à différents instants, et on représente de façon tridimensionnelle les profils obtenus des coefficients d'affaiblissement au moyen d'un système de traitement d'image connu en lui-même.

2. Procédé selon la revendication 1, caractérisé en ce que le rayonnement infrarouge est axialement focalisé en intensité et le foyer d'intensité du dispositif rayonnant en surface s'élève à un tiers du diamètre d'un dispositif rayonnant en surface rayonnant de forme annulaire.

3. Procédé selon la revendication 1, caractérisé en ce que l'intensité du rayonnement des sources de lumière agencées comme dispositif rayonnant en surface est modifiée à des valeurs jusqu'à $100 \mathrm{m}\, W \cdot cm^{-2} \cdot sr^{-1}$.

4. Procédé selon la revendication 1, caractérisé en ce que le pilotage des sources de lumière infrarouge s'effectue par multiplexage dans le temps, dans l'espace et suivant les longueurs d'ondes, et les éléments de volume sont mesurés de façon multidimensionnelle et différemment suivant les directions x, y et z par rapport aux éléments de volume voisins suivants.

5. Procédé selon la revendication 1, caractérisé en ce que la réception de la lumière diffusée transmise s'effectue axialement.

6. Procédé selon la revendication 1, caractérisé en ce que les spectres infrarouge des substances moléculaires existantes dans l'objet non homogène sont mesurés et résolus dans leur plage d'oscillation supérieure, et représentés dans la plage NIR.

7. Procédé selon la revendication 1, caractérisé en ce que pour la représentation sélective d'éléments de volume on modifie la section transversale du rayonnement.

8. Procédé selon la revendication 1, caractérisé en ce que pour la représentation contrastée de certaines structures in situ on utilise certains gradients d'affaiblissement spécifiques à l'élément de volume.

9. Procédé selon la revendication 1, caractérisé en ce que la différence entre des valeurs de flux volumique de particules qui se modifient par phase temporelle et celles qui se modifient de façon proportionnelle à l'absorption de lumière est déterminée sur le plan fonctionnel comme différence entre le flux volumique systolique proportionnel et le flux volumique diastolique proportionnel des globules rouges du sang, ou bien comme différence des quotients :

$$\frac{\text{flux vol. des globules }_{systol.}}{\text{flux vol. du plasma }_{systol.}} - \frac{\text{flux vol. des globules }_{diastol.}}{\text{flux vol. du plasma }_{diastol.}} = \zeta.$$

10. Agencement pour mettre en oeuvre le procédé selon l'une quelconque des revendications précédentes en appliquant une mesure précise de lumière diffusée définie sur le plan local et en volume avec plusieurs sources et récepteurs de lumière infrarouge agencés les uns à côté des autres dans un plan x - y, qui forment un dispositif rayonnant en surface micro-structuré, comprenant :

- des organes pour agencer le dispositif rayonnant en surface sur un secteur de la surface de la structure tridimensionnelle à analyser, le rayonnement du dispositif rayonnant en surface étant émis en direction z et entrant dans la structure tridimensionnelle et sortant en direction opposée (direction z);
- des organes pour alimenter en énergie les sources de lumière infrarouge, de telle sorte qu'il s'effectue un fonctionnement par impulsions avec une fréquence d'impulsions successives prédéterminée de diodes infrarouge qui sont utilisées comme sources de lumière; - des organes pour déplacer pas à pas le dispositif rayonnant en surface sur la surface de la structure tridimensionnelle et pour mesurer le rayonnement rétrodiffusé associé dans l'espace plusieurs fois au même emplacement mais avec différentes intensités d'émission pour saisir différents secteurs de volume de la structure et pour détecter un coefficient d'affaiblissement;
- des organes pour mémoriser les coefficients d'affaiblissement obtenus et pour déterminer un profil de coefficients d'affaiblissement des éléments de volume par une mesure multiple ou répétée sous différents angles d'incidence et/ou avec différentes longueurs d'onde et/ou à différents instants, ainsi que pour représenter de façon tridimensionnelle les profils obtenus des coefficients d'affaiblissement au moyen d'un système de traitement d'image connu en lui-même.

11. Agencement selon la revendication 10, caractérisé en ce que les composants d'émission et de réception individuels de la matrice émettrice/réceptrice à grande surface présentent un diamètre de l'ordre de quelques millimètres à quelques centimètres.

12. Agencement selon la revendication 10, caractérisé en ce que les émetteurs et les récepteurs dans les composants microstructurés sont agencés à une distance de 10 à 20 µm.

13. Agencement selon la revendication 10, caractérisé en ce que des éléments rayonnants de différentes longueurs d'onde agencés vers le récepteur forment les éléments microstructurés du composant.

Fig. 1

I

VO

Fig. 2

Fig.3

Fig. 4